# EUROPEAN PATENT SPECIFICATION

(11) **EP 1 472 986 B1**
(45) Date of publication and mention of the grant of the patent: **19.08.2009**
(21) Application number: 03076255.3
(22) Date of filing: 29.04.2003
(51) Int. Cl.: A61B 19/00, A61F 9/007, A61F 9/013, A61B 17/32, A61B 17/322, A61B 17/326

(54) **Devices and methods for preparing Descemet's membrane**
Geräte und Verfahren zur Präparation einer Descemet-Membran
Dispositifs et procédés pour la préparation d'une membrane de Descemet

(43) Date of publication of application: 03.11.2004
(73) Proprietor: Medical Technology Transfer Holding B.V., 3071 MJ Rotterdam (NL)
(72) Inventor: Melles, Gerrit Reinold Jacob, 3071 MJ Rotterdam (NL)
(74) Representative: Prins, Adrianus Willem

(56) References cited:
- US-A- 6 139 559
- US-B1- 6 358 262

## Description

The invention relates to a devices for use in eye surgery, especially for creating a means for treatment of (1) corneal endothelial disorders, (2) corneal surface disease, (3) corneal defects, (4) corneal contour changes, (5) refractive errors of the eye, and (6) types of cellular transplantation other than corneal endothelium for which Descemet's membrane can be used as a carrier device.

### I. Corneal endothelial disorders

In the treatment of corneal endothelial disorders it is common practice to replace the central part of the cornea, including stroma, Descemet's membrane and endothelium. To this end a full-thickness, cylindrical part of the cornea is removed and replaced by a similar part from a donor eye, a so called full-thickness keratoplasty.

This known technique has the disadvantage that the donor part of the cornea has to be sutured into the peripheral cornea of the patient, resulting in disorders in at least the outer surface of the cornea. This may be detrimental to the patient. Moreover, this technique results in relatively severe trauma to the patient, such as suture related problems, ineffective wound healing and postoperative astigmatism.

It has been recognised that most of the corneal endothelial disorders could be treated by replacement of the Descemet's membrane together with the endothelium. To this end a technique has been developed in which the Descemet's membrane is removed through a sclerocorneal tunnel, together with said endothelium and a slice of the stroma on which the Descemet's membrane is carried. This is then replaced by a donor membrane with endothelium, on said slice of the stroma. The slice is cut from the stroma using a thin knife.

This technique has proven to lead to lesser trauma to the patient, especially since the sclerocorneal tunnel can be self sealing, obviating the necessity of sutures. However, since part of the stroma has to be removed, the technique is still not optimal.

This part of the present invention relates to devices for use in eye surgery, especially for the treatment of corneal endothelial disorders, with which the problems of the known techniques can at least be partly lessened. To this end a device according to the present invention is characterized by the features of claim 1.

US 6 358 262 and US 6 139 559 disclose surgical instruments for cutting tissue within an eye having a straight handle and a cutting blade which is angled relative to said handle. The cutting part of said blade is flat and straight and includes an angle with part of the blade which is attached to the handle.

### I.a. Device for excising Descemet's membrane from host cornea

With a device according to the present invention it has surprisingly been found that the Descemet's membrane can be removed from a human or animal eye, without the necessity of removing part of the stroma also. In use the tip and at least part of said intermediate part of a device according to the present invention can be inserted into the anterior chamber of said eye, after which the Descemet's membrane can gently be stripped off from the posterior stroma. The Descemet's membrane, carrying the endothelium, can then be removed through said tunnel, after which it can be replaced by a donor Descemet's membrane through the same tunnel. The tip being angled relative to the intermediate part allows gentle stripping off the Descemet's membrane from the stroma, minimizing the risk of cutting into either the stroma or the Descemet's membrane.

Inter alia the development of a device according to the present invention has lead to a novel method for treatment of corneal endothelial disorders, in which a sclerocorneal tunnel is provided in an eye, into the anterior chamber, after which the Descemet's membrane is loosened at the side opposite said tunnel, i.e. the 6 o'clock surgical position when the tunnel is made at the 12 o'clock surgical position, after which the Descemet's membrane is pulled gently toward the said tunnel, loose from the stroma, by peeling, after which the Descemet's membrane can be removed through said tunnel and can be replaced by a donor Descemet's membrane through the same tunnel.

Preferably a fluid, especially a gas and more preferably air is introduced into the anterior chamber, through the same or a second sclerocorneal stab incision. Use of a gas such as air will provide for sufficient distance between the Descemet's membrane and the iris for moving the device. Whereas the gas will lead to a diffraction of the light falling into the eye, providing sufficient contrast for viewing the Descemet's membrane. In addition, a thin glide, preferable 0.1 to 1 mm in thickness and 3-5 mm in width, with a reflective surface facing the surgeon is inserted into the anterior chamber of the eye through the tunnel incision. The reflective surface disperses the incoming light from the operating microscope, further enhancing the visualization of the Descemet's membrane. Furthermore, a vital dye, preferably azafloxin, basic blue (nil blue sulphate), bismarck brown, basic red (rhodamine 6G), bengal red, brillant crysyl blue, eosin, fluorescein, gentian violet, indocyanine green, janus green, methylene green, methylene blue, neutral red, trypan blue, and trypan red is injected into the anterior chamber prior to the excision of Descemet's membrane. Although it is common knowledge that a vital dye does not stain the living endothelial cells as present in the living eye, surpisingly the dye has been found to stain pathological areas in between the cells or Descemet's membrane in areas where the cells are absent. The blue hue created further enhances the visualization of Descemet's membrane. Particularly good results have been obtained using a vital dye which is capable of staining said tissue or a tissue component, without diffusing through said tissue. A particular suitable example of a vital dye which is capable of staining tissue or tissue component without diffusing through said tissue is trypan blue in a concentration between 0.01 and 1%, and most preferable between 0.02 and 0.1%.

As an alternative for a air and vital dye injection into the anterior chamber, combined with insertion of a reflective glide, the anterior chamber can be filled with a visco elastic. A common, transparent visco elastic does not allow visualization of Descemets membrane. However, it has been found that a visco elastic mixed with small air bubbles, preferably les than 1 mm in diameter, and/or a vital dye as mentioned above, most preferably trypan blue in a concentration between 0.001% and 0.1%, and most preferably between 0.002 and 0.01%, the visualization of Descemet's membrane is effectively enhanced.

A device according to the present invention preferably has a tip, handle and intermediate part being substantially collinear, such that in a top view they extend approximately in a straight line. In side view the handle and tip preferably both enclose an angle with the intermediate part, in opposite directions. This enables proper introduction into the anterior chamber through a sclerocorneal tunnel, without the surrounding tissue of the eye, eyelids or the like blocking said device, whereas the tip can be steered accurately.

The tip preferably has a length which is substantially shorter than the length of the intermediate part, for example one-third to one-tenth thereof, so that the tip can be manoeuvred inside the anterior chamber, the intermediate part being of sufficient length to reach the ends of the Descemet's membrane. The tip preferably has a semi-sharp, semi-cutting edge, for example rounded, further minimising the risk of cutting into the stroma. The tip and intermediate part preferably are relatively small in width, such that they can be introduced through a sclerocorneal tunnel, for example less than 5 mm, preferably less than 4 mm and more preferably 3 mm or less, preferably having a smooth surface all around.

A device according to the present invention can have means for during use introducing a fluid, or preferably a gas such as air near the tip, that is into the anterior chamber, for the above mentioned purpose. Alternatively a separate means can be used for introduction of said fluid, for example through a different sclerocorneal incision. Integration of the said means in said device has the advantage that they can be handled simultaneously, providing for freedom of handling to the operator.

The device can be made of a material that can be resterilized, or the entire device or the intermediate part and the tip can be made of a disposable material.

### I.b. Device for harvesting Descemet's membrane from donor cornea or lens capusule from donor lens

The present invention furthermore relates to a set of a device according to the invention and a holding device according to claim 13.

Such holding device has the advantage that a Descemet's membrane can easily be removed from an corneoscleral donor button positioned onto the holding device with the epithelial side downward, and fixated onto said holding device by suction, in order to harvest and prepare a Descemet's membrane for transplantation.

### I.b.1. Harvesting Descemet's membrane with an intact endothelial cell layer from donor cornea

Preferably means are provided for submerging said donor button, at least the Descemet's membrane in a fluid, protecting said membrane and the endothelial cells it is carrying, and leading to the effect that the membrane will roll up without damaging the endothelial cells, because the membrane is afloat and a fluid film is surrounding the tissue protecting it against mechanical damage or sticking to itself. After staining the such roll with a vital dye, preferably trypan blue, the roll can be sucked into a injector used for introducing the membrane with an intact, living donor endothelial cell layer through a sclerocorneal tunnel incision into the host eye. Within the host anterior chamber, the roll can be unfolded by stretching the tissue over the iris, with or without using a viscoelastic, and the membrane is lifted upward against the host posterior stroma by injecting an air bubble underneath, i.e. at the endothelial side of the membrane.

With a holding device according to the present invention preferably a punch or other cutting device is used for excision of a rounded Descemet's membrane tissue piece, preferably 1 to 12, and most preferably 7 to 9 mm in diameter. Such a round tissue piece is produced by loosening the Descemet's membrane at the sceral spur, i.e. the anatomical outer edge of the membrane, with a knife or forceps, to within 3 to 4 mm from the corneal center. Then the membrane is positioned again in its anatomical position, and using a punch trephine, a partial thickness trephination is made through at least the Descemet's membrane and into the underlying stroma, resulting in a score line which is substantially closed in itself. Because the outer part of the Descemet's membrane within said score line had already been loosened, a rounded Descemet's membrane tissue piece can then easily be removed without the risk of tearing or fraying the membrane or damaging the endothelial cell layer.

### I.b.2. Harvesting Descemet's membrane with an intact endothelial cell layer and a stromal support layer from donor cornea

The device can also be used to harvest an intact, rounded Descemet's membrane tissue piece with a living endothelial cell layer, and with a thin layer of posterior corneal stroma. For this purpose, the donor button is incised in the area of the scleral spur, and a superficial dissection is performed to separate the posterior corneal layers (thin layer of posterior stroma, the Descemet's membrane and the endothelial layer) from the anterior corneal layers, across the cornea up to the scleral spur over 360 degrees. The dissected posterior layers are preferably 50 to 200 microns in thickness.

### I.b.3. Harvesting denuded Descemet's membrane from donor cornea

The device can also be used to harvest an intact, rounded Descemet's membrane tissue piece denuded of endothelium, for example as a carrier for heterologous endothelial cells or other cell types subsequently seeded onto the membrane. For this purpose, the Descemet's membrane is excised from the donor cornea as described in I.b.1. followed by mechanical or chemical removal of the cells, for example by dehydrating and hydrating the tissue through a graded series of alcohol.

### I.b.4. Harvesting denuded lens capsule from donor crystalline lens

The device can also be used to harvest an intact, rounded anterior or posterior lens capsule tissue piece denuded of lens cells, for example as a carrier for heterologous endothelial cells or other cell types subsequently seeded onto the membrane. For this purpose, the crystalline lens is removed from the donor eye and positioned with the posterior capsule downward onto the holding device, and fixated by suction. The anterior lens capsule is then trephinated from the crystalline lens, followed by mechanical or chemical removal of the cells, for example by dehydrating and hydrating the tissue through a graded series of alcohol.

### II. Corneal surface disease

In the treatment of corneal surface disease, and in particular persistent epithelial defects, it is common practice to prescribe topical medication or to perform mucosal membrane- and amniotic membrane transplantation. To this end, a donor membrane is sutured over the defect to allow the corneal epithelium to grow over the defect using the membrane as a scaffold. Disadvantages of these techniques are that the membranes retract after transplantation, so that the transplanted surface area decreases in diameter, and that the membranes tend to dissolve within months.

This part of the present description relates to the use of Descemet's membrane or lens capsule as a device in eye surgery for use as bandage tissue to cover an epithelial defect, i.e. as a scaffold for the host epithelium to grow over the defect. To this end a Descemet's membrane can be harvested, separated from the eye, in a condition to be transplanted into a human or animal eye. A denuded Descemet's membrane or lens capsule can be harvested as described in I.b.3 and I.b.4. The tissue piece, preferably 4-9 mm in diameter, is then positioned over Bowman's layer or the de-epithelialized corneal defect, with or without suture fixation.

### III. Corneal or lenticular defects

In the treatment of corneal or lenticular defects, it is common practice to perform mucosal membrane- and amniotic membrane transplantation. To this end, a donor membrane is sutured over the defect to allow the corneal epithelium to grow over the defect using the membrane as a scaffold. Disadvantages of these techniques are that the membranes retract after transplantation, so that the transplanted surface area decreases in diameter, and that the membranes tend to dissolve within months.

This part of the present description relates to the use of the Descemet's membrane or lens capsule in eye surgery for use as tissue replacements to fill a corneal or lenticular defect. To this end a Descemet's membrane can be harvested, separated from the eye, in a condition to be transplanted into a human or animal eye. A denuded Descemet's membrane or lens capsule can be harvested as described in I.b.3 and I.b.4. The tissue piece, preferably 1-9 mm in diameter is then positioned over the defect with suture fixation. The defect may be filled with multiple layers of the Descemet's membrane or lens capsule tissue.

In lenticular tissue defects, the Descemet's membrane or lens capsule can be used to seal the defect, for example a capsulorhexis opening.

### IV. Corneal contour changes

In the treatment of keratoconus and kerectasia it is common practice to replace the central part of the cornea, including stroma, Descemet's membrane and endothelium. To this end a, cylindrical part of the cornea is removed and replaced by a similar part from a donor eye, in a so full-thickness or lamellar keratoplasty.

This known technique has the disadvantage that the donor part of the cornea has to be sutured into the peripheral cornea of the patient, resulting in disorders in at least the outer surface of the cornea. This may be detrimental to the patient. Moreover, this technique results in relatively severe trauma to the patient, such as suture related problems, ineffective wound healing and postoperative astigmatism.

This part of the present description relates to the use of the Descemet's membrane or lens capsule in eye surgery for use as a tissue reinforcement device, especially for the treatment of corneal contour disorders, with which the problems of the known techniques can at least be partly lessened. To this end a Descemet's membrane can be harvested, separated from the eye, in a condition to be transplanted into a human or animal eye, which carries a thin layer of posterior stroma. In corneal contour changes, such as in keratoconus or kerectasia, the Descemet's membrane or lens capsule can be used as splint inside the host corneal stroma to reinforce the stroma. A Descemet's membrane with a thin layer of posterior stroma as described in I.b.2, or a denuded Descemet's membrane or lens capsule as described in I.b.3 and I.b.4. can be harvested. The tissue piece, preferably 7-12 mm in diameter is then positioned inside the cornea with or without suture fixation. Multiple layers of the Descemet's membrane or lens capsule tissue can be used to titrate the corneal curvature, i.e. to strengthen the cornea to a near physiological situation.

### V. Refractive error of the eye

In the treatment of refractive errors of the eye, it is common practice to perform biological or synthetic lenticle implantation. To this end, collagen or polymer lenses can be placed under the corneal epithelium or within the stroma, or in front of the crystalline lens. Disadvantages of these techniques are that these synthetic lenses lack long-term biocompatibility or may damage the intraocular structures.

This part of the present invention relates to the use of the Descemet's membrane or lens capsule in eye surgery for use as biological lenticular tissue additions to fill correct any refractive error of an eye. To this end a Descemet's membrane can be harvested, separated from the eye, in a condition to be transplanted into a human or animal eye, which membrane is ablated. With refractive errors of the eye, such as myopia, hyperopia and astigmatism, the Descemet's membrane or lens capsule can be used as lenticular tissue piece to change the refractive power of the eye. A denuded Descemet's membrane or lens capsule can be harvested as described in I.b.3 and I.b.4. The tissue piece, or multiple layers of tissue pieces, if necessary after collagen crosslinking to attach the layers, preferably 4 to 10 mm in diameter, is/are then positioned onto Bowman's membrane or underneath the corneal epithelium, inside the corneal stroma, or onto the anterior or posterior lens capsule. Before or after implantation, the (multilayered) tissue piece is ablated with an excimer laser or other device to create a lenticular shaped tissue piece.

### VI. Other cellular defects

Within the eye or human body, several types of cellular transplantations can be made feasible with the use of the Descemet's membrane or the lens capsule as a carrier device. A denuded Descemet's membrane or lens capsule can be harvested as described in I.b.3 and I.b.4. Most cell types can be seeded onto the membrane or capsule for subsequent transplantation. Because both the Descemet's membrane or the lens capsule are transparent, such a carrier device is particularly suitable for implantation throughout the eye, for example in the vitreous cavity, or on or underneath the retina.

A Descemet's membrane as described above, prepared for transplantation and separated from the cornea, enables less traumatic eye surgery.

The invention furthermore relates to a method for removing a Descemet's membrane from a human or animal eye, according to claim 17.

Such method allows controlled removal of the Descemet's membrane without substantial interference with the stroma, providing for a basis for Descemet's membrane with endothelium transplantation.

In this application Descemet's membrane has to be understood as at least encompassing parts of such membranes relevant for transplantation uses as discussed, and two or more of such membranes or said parts thereof at least partly overlapping

In the further subclaims preferable embodiments of the present invention are disclosed.

For a better understanding of the present invention embodiments thereof are shown and discussed hereafter, referring to the drawing and examples. The drawing show:
fig. 1 in side view a device according to the present invention;
fig. 2 in top view a device according to fig. 1;
fig. 2A-C in side, bottom and top view an embodiment with sizes indicated, by way of example only;
fig. 3 in top and side view an alternative embodiment of part of a device according to the present invention;
fig. 4 in side view a further embodiment of a device according to the present invention;
fig. 5 schematically in side view a device according to fig. 1 in an eye, during removal of the Descemet's membrane;
fig. 6 schematically in cross section a holding device according to the present invention;
fig. 7 a-d a holding device according to the present invention, during use;
fig. 8 a-f various embodiments of eyes with transplants according to the present invention; and
fig. 9 - 12 showing pictures of experiments with methods according to the present invention, wherein in

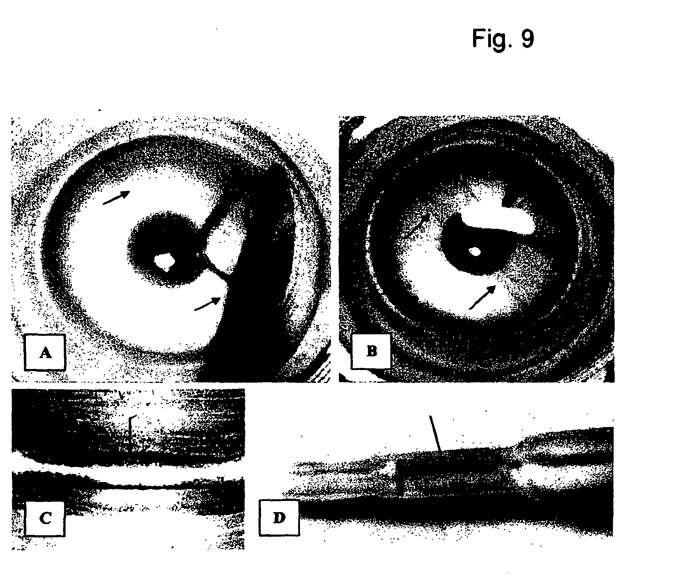

   - Figure 9:: Preparation of the 'donor' DM. (a) After partial trephination of the posterior side of a corneo-scleral button, a circular portion of DM (arrows) is stripped from the button with fine forceps. (b) After complete detachment, a Descemet-roll (arrows) forms spontaneously, with the endothelium on the outside. (c) To better visualize the donor tissue, the donor DM (arrow) is stained with trypan blue. (d) The Descemet-roll (arrow) is then sucked into an injector.

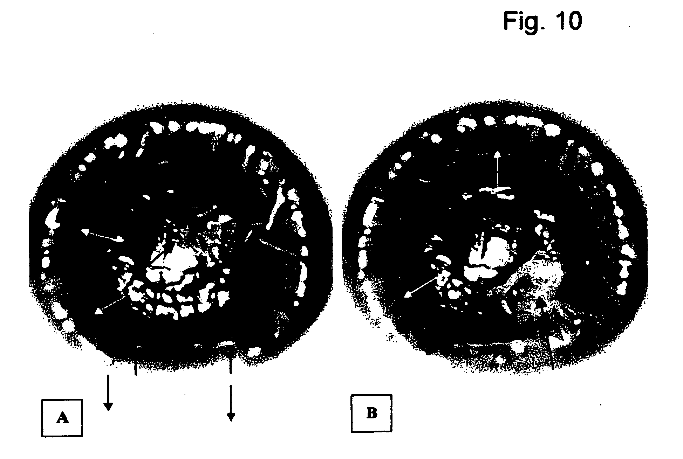

   - Figure 10:: Intraoperative pictures of the 'recipient' eye. (a) After filling the anterior chamber with air, through a scleral incision (black arrows) a Descemeto-rhexis is performed, i.e. a circular portion of DM (white arrows) is stripped from the posterior stroma. The green arrows point to the leading edge of the Descemeto-rhexis. (b) The Descemeto-rhexis has been completed over 360°, and the excised membrane is visible on top of the cornea (red arrow).

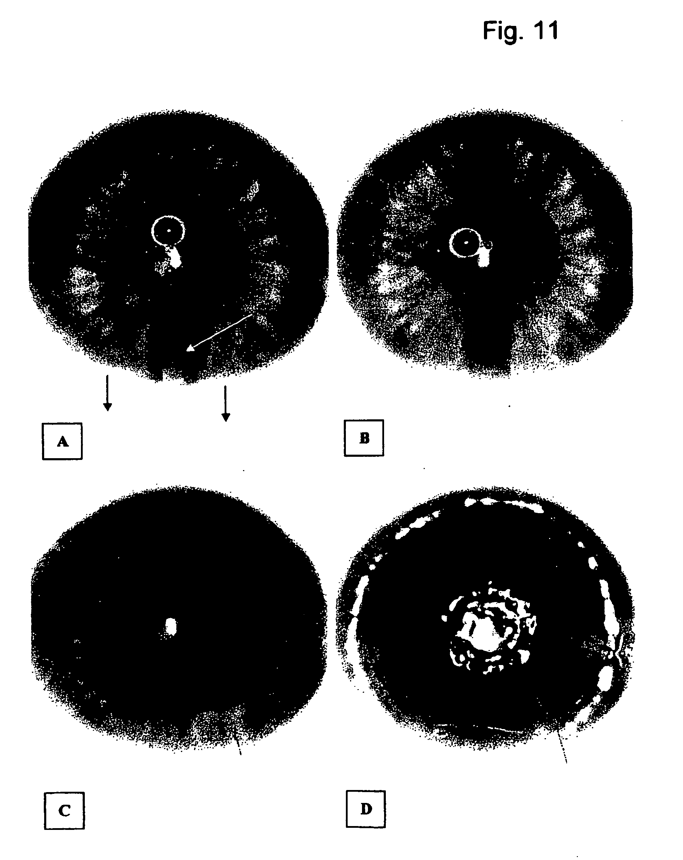

   - Figure 11:: Intraoperative pictures of the implantation of the 'donor' DM in the 'recipient' eye. (a) Through the scleral tunnel incision (black arrows), the donor DM (white arrow) is brought into the anterior chamber with the injector. (b) The Descemet-roll (green arrow) is positioned long the vertical meridian of the eye. (c) By injection of balanced salt solution onto the anterior side of the donor DM tissue, the membrane (green arrow) is unfolded and spread out over the iris. (d) With a blunt canula, an air bubble is than injected underneath the donor DM, to position the membrane (green arrow) against the recipient posterior corneal stroma.

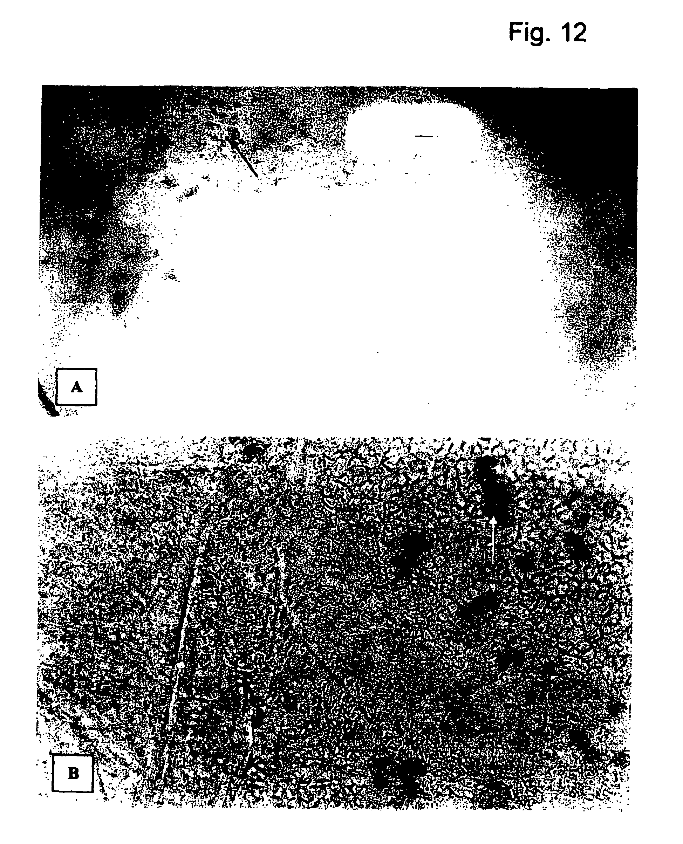

   - Figure 12:: The posterior corneal surface immediately after transplantation of DM in a human eye bank eye. (a) The donor endothelial cell layer is intact, with dispersed aeas of focal cell damage (black arrow). The blue background staining is an artefact caused by trypan blue staining of the anterior cornea. (b) At higher magnification, small clusters of damaged cells (white arrow) are visible in an intact endothelial cell layer. {Trypan blue and Alizarin red S; original magnification x25 (a) and x145 (b)}.

In the following description identical or similar parts are indicated with identical or similar reference signs. The embodiments shown are only shown by way of example and should not be interpreted as limiting the scope of protection.

### Device I: Device for excising Descemet's membrane from host cornea

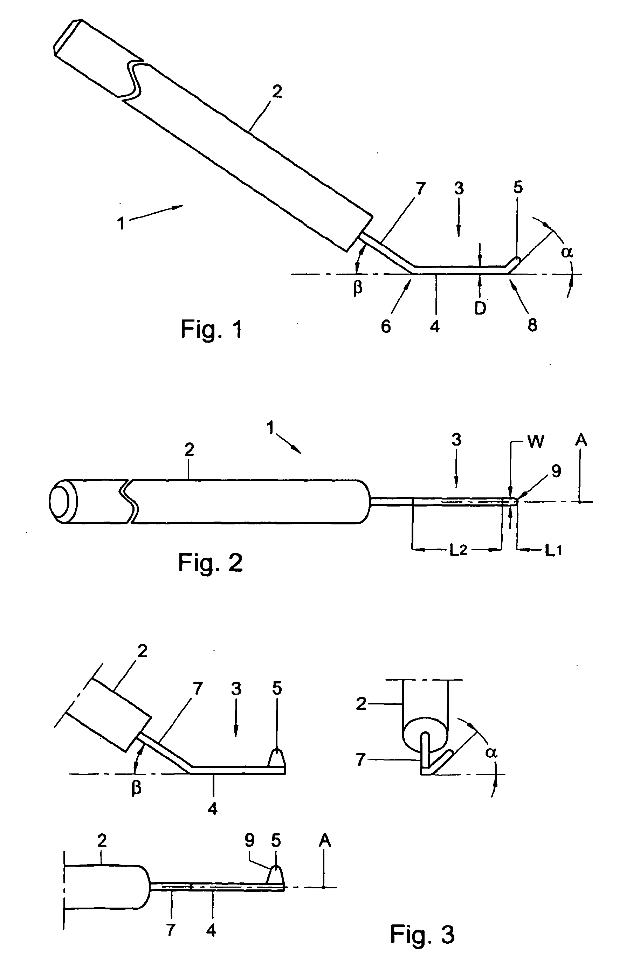

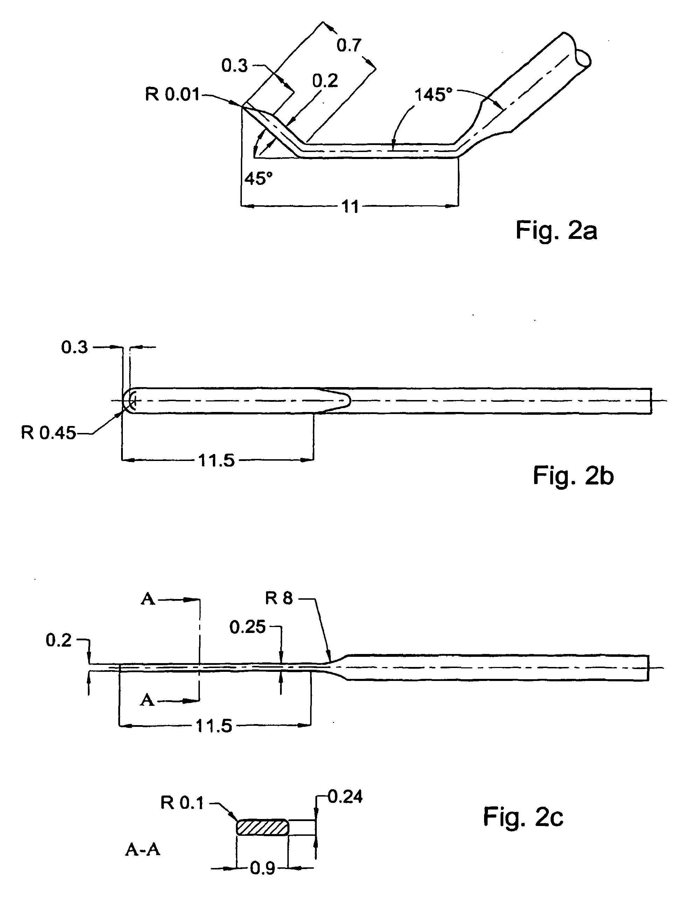

Fig. 1 and 2 show in side and top view a device 1 for use in eye surgery. This device comprises a handle 2 and an end part 3 extending there from. The handle 2 has been shown straight by way of example but can be bent or curved for better grip, ergonomics or aesthetic reasons. The handle 2 and end part 3 have in the embodiment shown in top view a common axis A and are therefore at least substantially collinear. The end part 3 is relatively thin with respect to the handle. The handle 2 and/or the end part 3 can be built from a re-usable material, such as surgical steel, or a disposable material, such as surgical plastic. The end part 3 is preferably made of a reflective material to provide better visualization of the host Descemet's membrane, by scattering incoming light from an operating microscope positioned above the eye.

The end part 3 comprises an intermediate part 4, a tip 5 and a connecting part 7. The intermediate part 4 is shown relatively straight and connects at the handle side 6 to the connecting piece 7 extending into the handle 2, whereas at the opposite side 8 it connects to the tip 5. In the embodiment shown the end part 3 is made in one piece, together with the connecting part 7, out of metal strip, such as surgical steel, bent to form the various parts. The end part 3 is preferably moulded in, glued in or otherwise attached to the handle 2 but could also be detachable from the handle, such that it can be changed or replaced by a different end part 3. The width W of the end part 3, at least of the intermediate part 4 and tip 5 is small, for example less than 5 mm, more specifically about 3 mm or less, the thickness D being even smaller, for example 1.0 to 2.5 mm or less. The length L₁ of the tip is small in relation to the length L₂ of the intermediate part, for example between one-third to one-tenth of said length L₁ or less.

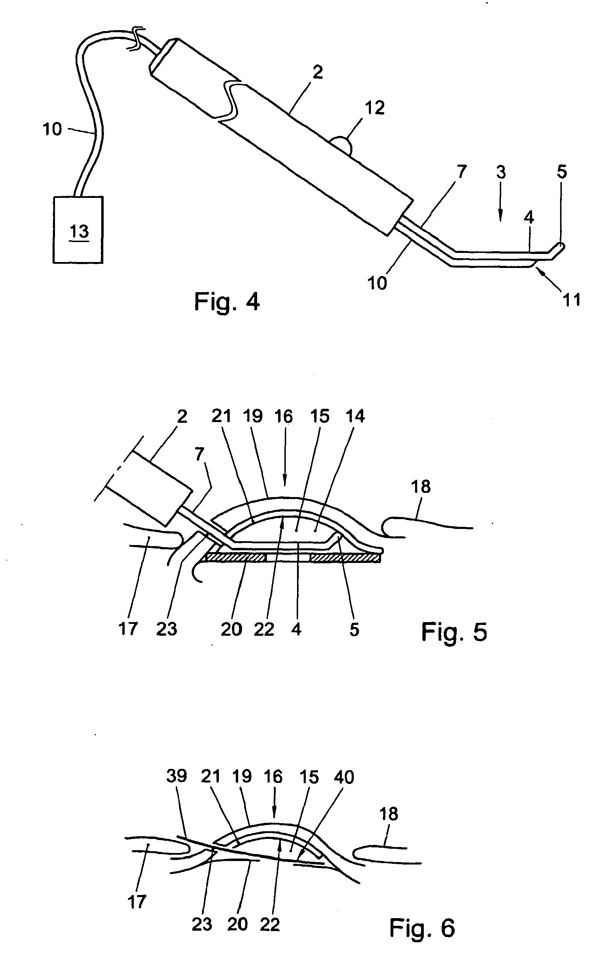

The tip 5 and connecting element 7 both enclose an angle α and β respectively with the intermediate part 4. The angle α is chosen between 10 and 80 degrees, more specifically between 25 and 65 degrees. An angle α between 35 and 55 degrees has shown preferable. In the embodiment shown said angle α is approximately 45 degrees. It is preferred that the angle β is comparable to the angle α although this can be chosen to comfort. The tip 5 has a rounded edge 9 which is semi-sharp, that is non cutting at normal circumstances. The angle α is chosen such that the device can be moved in axial direction with the end part 3 extending into the anterior chamber of the eye, as shown in fig. 5, without the risk of unintended damage to the intraocular structures of the eye.

By way of example, in an exemplary embodiment, fig. 2a-c show actual sizes of an embodiment, in side, bottom and top view. These should by no means be understood as limiting the scope of protection sought.

In fig. 3 the end part 3 and part of the handle 2 are shown of an alternative embodiment, in side view (top), top view (bottom) and front view (right handside). In this embodiment the tip 5 extends to the side of the intermediate part 4, for example at right angles. Also two tips could be provided, on opposite sides. This embodiments allows easy sideways peeling.

Fig. 4 shows a further embodiment of a device 1 according to the present invention, which is basically comparable to the embodiment of fig. 1. However, in this embodiment a tube 10 extend through or along the handle 2 towards the tip 5, ending close to the tip 5 in an outlet opening 11. The opposite end of the tube 10, which is flexible, is connected to a fluid supply 13, for example a syringe or a pump, especially for injecting air. On the handle an operating element 12 is provided for opening or blocking the outlet opening 11 or at least for letting air pass through the opening 11 or preventing such. This enables easy operation of the air supply by the surgeon, with one hand. Alternatively the supply 13 and tubing 10 with opening 11 could be mounted on the side of the device facing the surgeon. Alternatively the supply 13 and tubing 10 with opening 11 could be separate from the device 1, for example as shown in fig. 1, for separate introduction into an eye. With the supply 13 an air bubble 14 as shown in fig 5 can be provided in the anterior chamber 15 of an eye 16.

In fig. 5 schematically an eye 16 is shown, in cross section, for example approximately parallel to the main body plane. The upper eyelid 17 and lower eyelid 18 are shown, at both sides of the eye 16. Furthermore the cornea 19 and the iris 20 are shown, defining the anterior chamber 15. The cornea 19 is covered on the inside, facing the iris 20, by the Descemet's membrane 21 carrying the endothelium 22 to be removed. In the cornea 19 a sclerocorneal tunnel 23 is provided, preferably having a limited width of 5 mm or less, such that it is self sealing. The end part 3 of the device 1 is introduced through said tunnel 23 into the anterior chamber 15, tip 5 forward. As is shown in fig. 5, the angles α and β allow easy access, negotiating around the upper eyelid 17, wherein the intermediate part 4 can be held approximately parallel to the iris 20 and the tip 5 can be forced gently against the Descemet's membrane 21. The Descemet's membrane can than be stripped off into the direction of the tunnel 23 by pulling at different parts of the outer or leading edge of the membrane 21. After loosening, the membrane 21 will be rippled and can ultimately be pulled out of the anterior chamber 15 without substantial damage to the stroma (not shown in fig. 5) of the host cornea. A donor Descemet's membrane 21 can then be introduced into the anterior chamber 15 of the eye 16 for transplantation.

### Device II: Device for visualization of Descemet's membrane

Fig. 6 shows a device 39 for use in eye surgery. This device is made of a flexible, semi-stiff material, such as a surgical plastic, 2 to 5 mm in width, and 0.1 to 1 mm in thickness. The device has a round tip to avoid damage to the intraocular structures, and is at least 20 mm in length to allow for better grip, ergonomics or aesthetic reasons. Both surfaces or at least the surface facing the surgeon is relflective, so that the incoming light from an operating microscope positioned above the eye, is reflected by the device. The device can be built from a re-usable material, such as surgical steel, or a disposable material, such as surgical plastic.

During surgery, the device is introduced into the anterior chamber 15 of the eye through the sclerocorneal tunnel 23 incision, and rests on the iris 20. The reflective surface of the device disperses or scatters the incoming light, and thereby facilitates the visualization of Descemet's membrane.

In fig. 6 schematically an eye 16 is shown, in cross section, for example approximately parallel to the main body plane. The upper eyelid 17 and lower eyelid 18 are shown, at both sides of the eye 16. Furthermore the cornea 19 and the iris 20 are shown, defining the anterior chamber 15. The cornea 19 is covered on the inside, facing the iris 20, by the Descemet's membrane 21 carrying the endothelium 22 to be removed. In the cornea 19 a sclerocorneal tunnel 23 is provided, preferably having a limited width of 5 mm or less, such that it is self sealing. The device 39 is introduced through said tunnel 23 into the anterior chamber 15. The surface of the device 39, or at least the surface 40 facing the surgeon is made of a reflective material, to facilitate the visualization of the Descemet's membrane.

By way of example, in an exemplary embodiment the device is 20 mm in length, 4 mm in width and 0.2 mm in thickness, and made of aluminum coated paper. These should by no means be understood as limiting the scope of protection sought.

### Device III: Holding device for excising donor Descemet's membrane and donor lens capsule

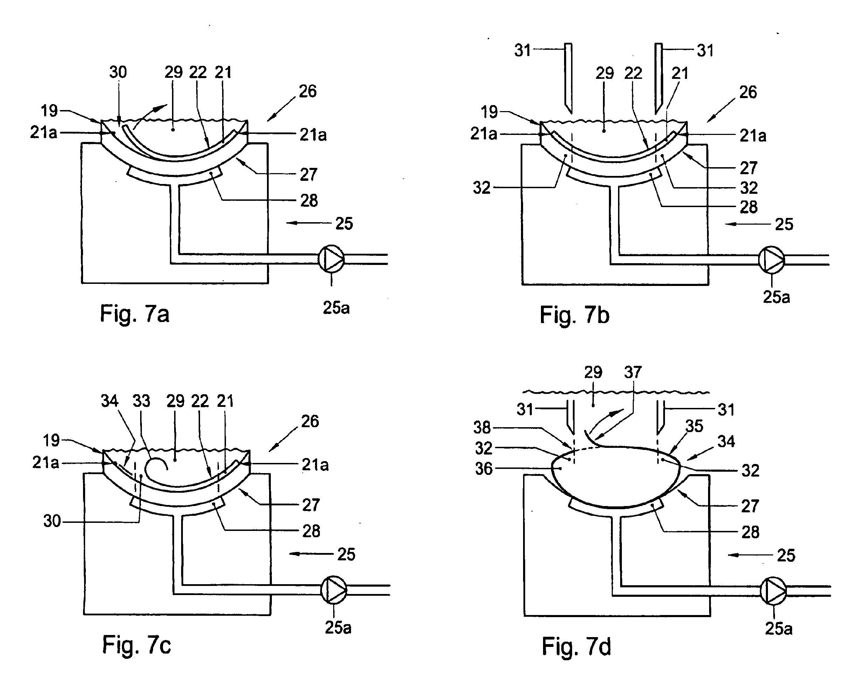

In fig. 7 a-c holding device 25 is shown, carrying a donor corneoscleral button 26, the Descemet's membrane 21 with the endothelium 22 facing upward. At least in the button 26 a fluid is provided, preferably a physiological compatible balanced salt solution 29, submerging the membrane 21. Alternatively the holding device 25 itself can be submerged. The holding device 25 is provided with a bowl shaped element 27 wherein the convex or epithelial side of the button 26 rests. At the bottom of said bowl shaped element a suction pad 28 is provided, with suction means 25a for providing a slight under pressure in said pad 28, holding the button 26 gently but stable.

Preferably the suction pad 28 is 4 to 13 mm and most preferably 8 to 9 mm in width, and 0.5 to 3.0 mm and most preferably 2.0 mm in depth. The bowl shaped surface 27 of the holding device 25 is made of a reflective material, so that the incoming light of an operating microscope positioned above the holding device, is reflected by the bowl shaped surface 27 through the transparent cornea 19, enhancing the visualization of the Descemet's membrane 21. Alternatively, the complete holding device can be made of a transparent material with a light source from underneath. The visualization of the donor Descemet's membrane can be further improved by using a vital dye as described before.

In this position the Descemet's membrane can be removed as follows. Firstly at one side (in fig. 7 at the left) the Descemet's membrane is loosened by pulling up slightly from the stroma 19, providing for a gap 30. Then an incision 32 is made, for example using a punch 31, schematically shown, which incision 32 goes through the Descemet's membrane 21 and into but not through the stroma 19 and/or cornea 26. The incision 32 is closed in itself, defining an inner part 33 and a peripheral part 34. The incision extends at least partly through said gap 30, providing that at least a small part of the inner part 33 of the Descemet's membrane 21 is free from the stroma 19. Then the said part lying free can be pulled gently, freeing the further membrane 21, which will roll up, as shown in fig. 7c. The Descemet's membrane 21 can then be stored and be used for transplantation.

In fig. 7d holding device 25 is shown, carrying a donor lens 34, consisting of a lens capsule 35 and lens mass 36. The Descemet's membrane 21 with the endothelium 22 facing upward. The entire holding device is preferably submerged in a physiological compatible balanced salt solution 29, submerging the lens capsule 35. The lens rests on the bowl shaped element 27 wherein the convex or epithelial side of the button 26 rests. At the bottom of said bowl shaped element a suction pad 28 is provided, with suction means 25a for providing a slight under pressure in said pad 28, holding the lens 34 gently but stable.

In this position the lens capsule can be removed as follows. An incision 32 is made, for example using a punch 31, schematically shown, which incision 32 goes through the lens capsule into the lens mass, but not through the lens 34. The incision 32 is closed in itself, defining an inner part 37 and a peripheral part 38. Then the central part 37 can be pulled gently from the lens mass, freeing the further lens capsule 37, which will roll up. The lens capsule can then be stored and be used for transplantation.

### Device IV: Rounded Descemet's membrane tissue piece with an intact, living, autologous endothelial cell layer

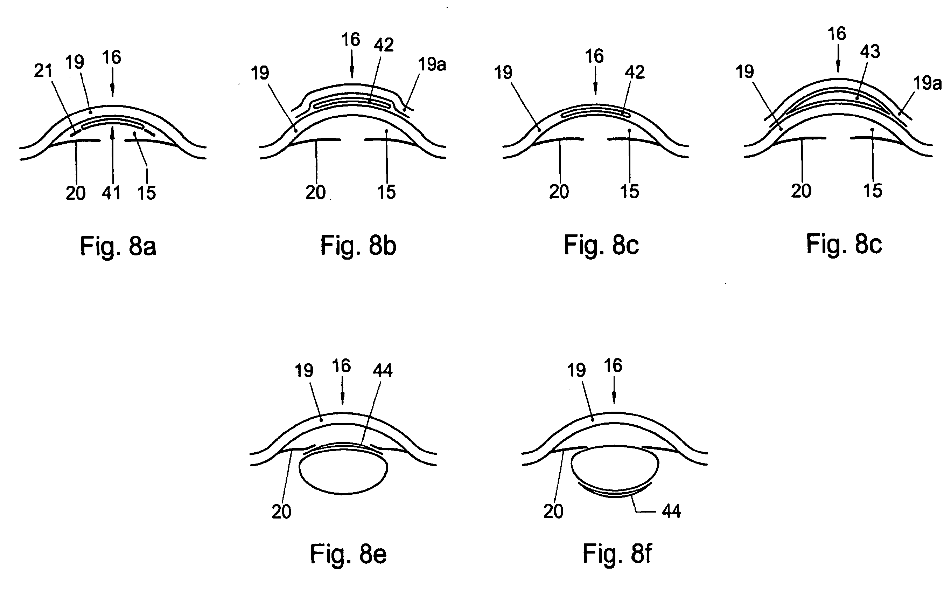

Fig. 8a shows a device for use in eye surgery. This device consists of a rounded Descemet's membrane tissue piece with an intact, living, autologous endothelial cell layer. The tissue piece is 7 to 9 mm in diameter, and may vary in thickness up to 20 to 50 microns. The device may be stained with a vital dye as described before, to facilitate its visualization.

By way of example, in an exemplary embodiment the device is 9 mm in diameter, and used for transplantation by positioning the device against the host posterior stroma from which the host Descemet's membrane has been stripped off. In fig. 8a schematically an eye 16 is shown, in cross section, for example approximately parallel to the main body plane. Furthermore the cornea 19 and the iris 20 are shown, defining the anterior chamber 15. The cornea 19 is covered on the inside, facing the iris 20, by the Descemet's membrane 21. The device 41 is centered and positioned against the host cornea 19. These should by no means be understood as limiting the scope of protection sought.

### Device V: Rounded Descemet's membrane- or lens capsule tissue piece denuded of cells or cellular material

Fig. 8b shows a device for use in eye surgery. This device consists of a rounded Descemet's membrane- or lens capsule tissue piece. The tissue piece is 1 to 12 mm in diameter, and may vary in thickness up to 20 to 50 microns. The device may be stained with a vital dye as described before, to facilitate its visualization.

By way of example, in an exemplary embodiment the device is 9 mm in diameter, and used for transplantation by positioning the device onto the Bowman's layer of a de-epithelialized host cornea as a scaffold for the corneal epithelium to re-epithelialize the cornea. In fig. 8b schematically an eye 16 is shown, in cross section, for example approximately parallel to the main body plane. Furthermore the cornea 19 and the iris 20 are shown, defining the anterior chamber 15. The cornea 19 is covered on the outside by the corneal epithelium 19a. The device 42 is centered and positioned against the host anterior cornea 19, underneath the epithelium 19a.

In another exemplary embodiment the device is 9 mm in diameter, and used for transplantation by positioning the device (or multiple devices on top of each other) within an anterior corneal defect or lenticular defect, or within the stroma, to fill in a corneal defect. In another exemplary embodiment the device is 9 mm in diameter, and used for transplantation by positioning the device within a corneal pocket to reinforce the corneal stroma to induce a corneal curvature change. In fig. 8c schematically an eye 16 is shown, in cross section, for example approximately parallel to the main body plane. Furthermore the cornea 19 and the iris 20 are shown, defining the anterior chamber 15. The device 42 is centered and positioned inside the host cornea 19., i.e. within a stromal pocket.

In yet another exemplary embodiment the device is 9 mm in diameter, and used for transplantation by positioning the device in another part of the eye to fill in a cellular defect, in which the device is used as a carrier device to transplant one or multiple cell layers other than corneal endothelium, for example retinal or subretinal cells.

These examples should by no means be understood as limiting the scope of protection sought.

### Device VI: Rounded Descemet's membrane- or lens capsule (multilayered) tissue piece denuded of cells or cellular material, with a lenticular shape

Fig. 8d shows a device for use in eye surgery. This device consists of multiple layers of stacked, rounded Descemet's membrane- or lens capsule tissue pieces, if necessary attached to each other by collagen crosslinking. The device is 4 to 10 mm in diameter, and may vary in thickness up to 500 microns, but most preferably up to 100 to 200 microns. Laser molding of the device, for example excimer laser molding, may be performed at any time before the surgery or positioning the device onto or within the eye, or after positioning the device onto or within the eye. The device may be stained with a vital dye as described before, to facilitate its visualization.

By way of example, in an exemplary embodiment the device is 9 mm in diameter, and 150 microns in thickness. After removal of the epithelium of the host cornea, the device is positioned onto Bowman's layer and centered over the pupil. An excimer laser is used to ablate a peripheral or central area of the device, to create a lenticular shape of the device. Then the host epithelium is allowed to grow back over the device, so that the device is encompassed by the epithelium, i.e. located underneath the epithelium.

In fig. 8d schematically an eye 16 is shown, in cross section, for example approximately parallel to the main body plane. Furthermore the cornea 19 and the iris 20 are shown, defining the anterior chamber 15. The cornea 19 is covered on the outside by the corneal epithelium 19a. The device 43 is centered and positioned against the host anterior cornea 19, i.e. onto Bowman's layer underneath the epithelium 19a.

In another exemplary embodiment the device is positioned inside the corneal stroma, i.e. inside a stromal pocket, or underneath a corneal flap, for example a microkeratome flap. In yet another exemplary embodiment the device is positioned onto the anterior or posterior lens capsule.

In fig. 8e and 8f schematically an eye 16 is shown, in cross section, for example approximately parallel to the main body plane. Furthermore the cornea 19 and the iris 20 are shown. The device 44 consisting of a Descemet's membrane as discussed with respect to fig. 8a-d or such membranes or parts thereof, at least partly overlapping is centered and positioned onto the host anterior or posterior crystalline lens surface, i.e. onto the host anterior or posterior lens capsule.

These should by no means be understood as limiting the scope of protection sought.

The present invention is by no means limited to the embodiments shown and discussed here before. Many variations are possible within the scope of the invention as defined by the claims.

For example the shape and dimensions can be varied, depending on for example the eye to be operated. The tip can have a different shape and could be made of a material different from the intermediate part. The tip could for example be formed as a notch, roughened bulge or button shaped element on the intermediate part, having at least one surface enclosing an angle with said intermediate part, such that the Descemet's membrane can be pulled of from the stroma. These variations and any combination of features of the embodiments shown are concidered to be encompassed by the present invention.

A detailed example of the use of one of the embodiments described above is given below.

Posterior lamellar keratoplasty through a sclero-corneal incision may be an alternative procedure for penetrating keratoplasty, in the management of corneal endothelial disorders like pseudophakic bullous keratopathy, and Fuchs' endothelial dystrophy. If the near spherical, anterior corneal surface of the recipient is maintained, the best corrected visual acuity may not be reduced due to postoperative astigmatism. When the use of sutures is eliminated, postoperative suture-loosening and/or -infection can not occur. Late wound dehiscence, frequently encountered with full-thickness transplant wounds, may rarely occur with sclero-corneal tunnel incisions.

For transplantation of the corneal endothelium several experimental techniques have been described. Most authors used a technique in which cultured endothelium was grown onto a corneal button or synthetic carrier, and subsequently transplanted through a full-thickness transplant wound. McCulley et al evaluated the injection of endothelial cells into the anterior chamber. Barraquer et al, and Busin et al, have described transplantation of a posterior lamellar disc underneath a sutured anterior lamellar flap. Melles et al described a technique for posterior lamellar keratoplasty, in which an unsutured, posterior lamellar disc was transplanted through a 9.0 mm scleral incision.
Now a surgical technique for transplantation of Descemet's membrane and its endothelium through a sclero-corneal tunnel incision has been developed, to minimize postoperative astigmatism, and to eliminate suture-related problemes and complications due to ineffective wound healing. In human eye bank eyes, the feasibility of the procedure was evaluated using keratometry, endothelial vital and supravital staining, and light microscopy.

### MATERIALS AND METHODS

### Human cadaver eye model.

Thirty human cadaver eyes were obtained through Bio Implant Services, Leiden, and the Cornea Bank of the Netherlands Ophthalmic Research Institute, Amsterdam. Fifteen globes were used as 'recipient' eyes (eyes 1-15; Table 1). From 15 'donor' eyes (eyes 16-30; Table 1), corneo-scleral buttons were excised less than 36 hours post mortem, and stored by organ culture in modified minimum essential medium (EMEM) at 31° C. After two weeks, in which routine tests for endothelial cell morphology and viability were performed, donor Descemet's membranes were stripped from the buttons for transplantation to recipient eyes.

Each recipient globe was placed in an eye holder equipped with a suction ring, to immobilize the posterior globe and to control the intraocular pressure (IOP) (Schiøtz tonometer). Globes were oriented with the 12 o'clock anatomical position toward the surgeon. The epithelium was gently removed with a cellulose spounge. Corneas were dehydrated by subjecting the globes to an IOP of 50 to 60 mmHg at room temperature, for 30-60 minutes, until central thinning was achieved to 0.60 to 0.65 mm (Pach-pen XL, Mentor, Norwell, MA).

### Operative procedure.

Donor: Corneo-scleral buttons were mounted endothelial side up on a custom made holder with a suction cup, and the holder carrying the button was completely immersed in balanced salt solution. A superficial trephination was made into the posterior stroma with a 9.0 mm trephine (Ophtec, Groningen, NL). Descemet's membrane was stripped from the posterior stroma, so that a 9.0 mm diameter flap of posterior Descemet's membrane with its endothelial monolayer was obtained (Figure 9a). Due to the elastic properties of the membrane, a 'Descemet-roll' formed spontaneously, with the endothelium at the outer side (Figure 9b). The Descemet-roll was stained with trypan blue 0.06% (VisionBlue^{TM}, D.O.R.C. International, Zuidland, The Netherlands) (Figure 9c), and sucked into a custom made injector (Figure 9d).

*Recipient:* In eyes 1-15 (Table 1), an 9.0 mm diameter marker (Katena, Eye Care, Gorinchem, NL) was centered over the cornea. A 5.0 mm wide sclerocorneal tunnel incision was made with a slit-knife, that entered the anterior chamber just at the mark (Figure 10a). A circular portion of Descemet's membrane was scraped from the posterior stroma, so that a 9.0 mm diameter 'Descemeto-rhexis' was created, and the membrane was removed from the eye (Figures 10b).

Using the injector, the donor Descemet-roll was brought into the anterior chamber (Figure 11a), and oriented along the vertical meridian of the eye (Figure 11b). The inner portion of the Descemet-roll was gently manipulated with a canula, to spread out the donor Descemet's membrane over the iris of the recipient (Figure 11c). Then, the canula was placed underneath the membrane, and an air bubble was injected through the canula into the anterior chamber, so that the anterior chamber was completely filled with air, and the donor Descemet's membrane was lifted against the recipient posterior stroma (Figure 11d). If neccessary, the donor tissue was repositioned by pulling at the edge of the transplant with fine forceps.

### Keratometry.

In human recipient eyes 1-10 (Table 1), three consecutive keratometry readings (Javal-Schiotz ophthalmometer, Haag-Streit) were taken before and after surgery, and averaged for each corneal meridian. Statistical analysis was performed using the paired Student-t-test.

### Evaluation of endothelium.

After surgery, from human recipient eyes 11-15 corneo-scleral buttons were removed, that contained donor Descemet's membrane from eyes 26-30. Immediately after excision of the buttons, donor endothelial cell viability was evaluated as previously described.

### Microscopy.

Corneo-scleral buttons from recipient eyes (n=10) were fixed in 2.5% glutaraldehyde in 0.1 mol/L sodium cacodylate buffer (pH 7.3, 340 mOsm). One corneal half was dehydrated through a graded series of ethanol followed by intermediate changes of methylbenzoate and xylene, and embedded in paraffin. One micron sections were cut along the vertical meridian; sections were stained with hematoxylin eosin, and photographed (Vanox light-microscope, Olympus Optical Co LTD, Tokyo, Japan; Ektachrome 64 color slide film, Kodak).

### RESULTS

In recipient eyes 1-10, the astigmatic error averaged 0.7 D (sd ± 0.3 D) before, and 1.0 D (sd ± 0.6 D) after surgery (p>0.1).

Macroscopic evaluation of the posterior corneal surface of recipient eyes 1-15 showed Descemet's membrane transplants with complete apposition to the recipient posterior stroma. Before surgery, the corneo-scleral buttons from donor eyes 26-30 showed no detectable endothelial cell damage. After transplantation, the donor Descemet's membranes obtained from these eyes showed an intact endothelial cell layer, with dispersed, small clusters of damaged cells (Figures 12a and 12b). Across the entire donor posterior surface, the estimated percentage of endothelial cell damage was 3.4% (± 1.1%) .

Light microscopy of the excised recipient corneal buttons showed a complete apposition of the donor Descemet's membrane and endothelium to the recipient posterior stroma. In three eyes, the far corneal periphery showed overlapping of the recipient Descement's membrane by the donor Descemet's membrane.

### References

14. Pels E, Schuchard Y. Tissue storage. E: Organ culture and endothelial evaluation as a preservation method for human corneas. In: Brightbill FS (ed): Corneal surgery. Theory, technique, and tissue. St Louis, CV Mosby Co, 1986:93-102.
15. Melles GRJ, Wijdh RHJ, Cost B, Beekhuis WH, Binder PS, van Rij G, Groot K. Effect of blade configuration, knife action, and intraocular pressure on keratotomy incision depth and shape. Cornea 1993;12:299-309.

## Claims

1. Device (1) for use in eye surgery, comprising a handle (2) with at one end a scraping element (3), said scraping element (3) having a tip (5) and an intermediate part (4) between said connecting part (7) and said tip (5), said connection part (7) including an angle (6) with the intermediate part, **characterised by** said tip (5) including an angle (α) with a general direction of said intermediate part (4) between 10 and 80 degrees.

2. Device according to claim 1, wherein said angle (α) is between 25 and 65 degrees, more specifically between 35 and 55 degrees and preferably approximately 45 degrees.

3. Device according to claim 1 or 2, wherein the intermediate part (4) is substantially straight and in top view collinear with the handle (2), the tip (5) extending in the axial direction of said handle (2) and intermediate part (4).

4. Device according to any one of the preceding claims, wherein the length of the tip (5) between the intermediate part (4) and the opposite free end (9) of the tip (5) is substantially smaller than the length of the intermediate part (4) between the tip (5) and the connecting part (7), preferably no more than one third of said length, more preferably less than one fifth to one tenth.

5. Device according to any one of the previous claims, wherein the tip (5) has a semi-sharp edge (9), preferably rounded.

6. Device according to any one of the previous claims, wherein said tip (5) is bent in a first direction, relative to the intermediate part (4), the opposite side of at least the intermediate (4) part being smooth, preferably having rounded longitudinal edges.

7. Device according to any one of the previous claims, wherein the tip (5) and intermediate part (4) have a width less than 5 mm, more specifically less than 4 mm and preferably less than 3 mm.

8. Device according to any one of the previous claims, for removing an Descemet's membrane from an eye through an opening in an outer sphere of said eye, wherein said scraping element (3) in top view has a length between the bend (6) positioned at the connecting part (7) and the end (9) of the tip (5) which is smaller than the length of the anterior chamber (15) of a human eye.

9. Device (1) according to any one of the previous claims, wherein means (10-13) are provided for introducing a fluid, especially air near said tip (5), comprising a tubing (10) with an opening (11) near said tip (5), said tubing (10) being connectable to a fluid supply (13).

10. Device (1) according to claim 9, wherein an operating element (12) is provided for during use forcing said fluid through said tubing (10).

11. Assembly of a device (1) according to any one of claims 1- 8 and a device for introducing a fluid such as air in an anterior chamber (15) of an eye.

12. Set of a device (1) according to anyone of claims 1-10 or an assembly according to claim 11 and a visualization device (39), said visualization device comprising a flexible resilient end for introduction into an interior chamber of an eye through a sclero corneal tunnel, said end being provided with at least one light reflecting surface for reflecting light through the cornea of said eye.

13. Set of a device (1) according to anyone of claims 1-10 or an assembly according to claim 11 and a holding device for holding part of an eye, comprising part of a cornea and the Descemet's membrane, said holding device being provided with a bowl shaped part and a suction element in said bowl shaped element, such the said part of said eye can be positioned in said bowl shaped element, held by said suction element, the Descemet's membrane facing upward, away from said bowl shaped element.

14. Set according to claim 13, wherein means are provided for submerging at least said Descemet's membrane in a fluid.

15. Set according to claim 13 or 14, wherein said bowl shaped part is at least partly provided with a light reflecting surface.

16. Kit of parts comprising a set according to any one of claims 13-15 and a punching device for cutting a score line in said Descemet's membrane, said score line extending at a small distance from the outer edge of said Descemet's membrane.

17. Method of removing a Descemet's membrane from an eye outside the human or animal body, comprising the steps of:
- making at least one incision in or near the lens of said eye, into the anterior chamber;
- inserting the tip of a device according to anyone of claims 1-10 through said opening into said anterior chamber and pulling the Descemet's membrane towards said opening, loosening said membrane from the cornea or Stroma; and
removing said Descemet's membrane from said anterior chamber through said opening.

18. Method according to claim 17, further comprising the step of introducing a fluid, especially a gas, preferably air into said anterior chamber before or during loosening of said Descemet's membrane.

## Patentansprüche

1. Vorrichtung zur Verwendung in der Augenchirurgie, mit einem Griff (2), der an einem Endbereich ein Schabelement (3) aufweist, wobei das Schabelement (3) einen Endteil (5) und einen zwischen einem Verbindungsteil (7) und dem Endteil (5) angeordneten Zwischenteil (4) aufweist, wobei der Verbindungsteil (7) mit dem Zwischenteil einen Winkel (β) einschließt,
**dadurch gekennzeichnet, dass** der Endteil (5) mit einer generellen Richtung des Zwischenteils (4) einen Winkel (α) von 10 bis 80 Grad einschließt.

2. Vorrichtung nach Anspruch 1, bei der der Winkel (α) 25 bis 65 Grad, insbesondere 35 bis 55 Grad und vorzugsweise ungefähr 45 Grad beträgt.

3. Vorrichtung nach Anspruch 1 oder 2, bei der der Zwischenteil (4) im Wesentlichen gradlinig ist und in Draufsicht kollinear mit dem Griff (2) verläuft, wobei sich der Endteil (5) in der Axialrichtung des Griffs (2) und des Zwischenteil (4) erstreckt.

4. Vorrichtung nach einem der vorhergehenden Ansprüche, bei der die Länge des Endteils (5) zwischen dem Zwischenteil (4) und dem gegenüberliegenden freien Ende (9) des Endteils (5) wesentlich kürzer ist als die Länge des Zwischenteils (4) zwischen dem Endteil (5) und dem Verbindungsteil (7) und vorzugsweise höchstens ein Drittel der Länge und insbesondere weniger als ein Fünftel bis weniger als ein Zehntel beträgt.

5. Vorrichtung nach einem der vorhergehenden Ansprüche, bei der der Endteil (5) einen halbscharfen, vorzugsweise gerundeten Rand (9) aufweist.

6. Vorrichtung nach einem der vorhergehenden Ansprüche, bei der der Endteil (5) relativ zu dem Zwischenteil (4) in einer ersten Richtung gebogen ist, wobei die gegenüberliegende Seite mindestens des Zwischenteils (4) glatt ist und vorzugsweise gerundete Längsränder aufweist.

7. Vorrichtung nach einem der vorhergehenden Ansprüche, bei der der Endteil (5) und der Zwischenteil (4) eine Breite von weniger als 5 mm, insbesondere weniger als 4 mm und vorzugsweise weniger als 3 mm haben.

8. Vorrichtung nach einem der vorhergehenden Ansprüche zum Entfernen einer Descemet-Membran von einem Auge durch eine Öffnung in einem Außenrundungsbereich des Auges, wobei das Schabelement (3) in Draufsicht zwischen der an dem Verbindungsteil (7) gelegenen Krümmung (6) und dem Ende (9) des Endteils (5) eine Länge hat, die kürzer ist als die Länge einer menschlichen vorderen Augenkammer (15).

9. Vorrichtung nach einem der vorhergehenden Ansprüche, bei der zum nahe dem Endteil (5) erfolgenden Einführen eines Fluids, insbesondere Luft, Vorrichtungen (10-13) vorgesehen sind, die einen Schlauch (10) mit einer nahe dem Endteil (5) gelegenen Öffnung (11) aufweisen, wobei der Schlauch (10) mit einer Fluidzufuhr (13) verbindbar ist.

10. Vorrichtung nach Anspruch 9, bei der ein Betätigungselement (12) vorgesehen ist, um bei Betrieb das Fluid durch den Schlauch (10) zu drücken.

11. Baugruppe nach einem der Ansprüche 1 - 8 und einer Vorrichtung zum Einführen eines Fluids wie z.B. Luft in eine vordere Augenkammer (15).

12. Set aus einer Vorrichtung (1) nach einem der Ansprüche 1-10 oder einer Baugruppe nach Anspruch 11 und einer Visualisierungsvorrichtung (39), wobei die Visualisierungsvorrichtung ein flexibles elastisches Ende zur Einführung in eine Innenkammer eines Auges durch einen sklero-cornealen Tunnel aufweist, wobei das Ende mindestens eine lichtreflektierende Fläche zum Reflektieren von Licht durch die Cornea des Auges aufweist.

13. Set aus einer Vorrichtung (1) nach einem der Ansprüche 1-10 oder einer Baugruppe nach Anspruch 11 und einer Haltevorrichtung zum Halten eines Teils des Auges, der einen Teil einer Cornea und die Descemet-Membran umfasst, wobei die Haltevorrichtung mit einem schalenförmigen Teil und einem in dem schalenförmigen Teil angeordneten Saugelement versehen ist, derart, das der Teil des Auges in dem schalenförmigen Teil positioniert werden kann, wobei der Teil des Auges von dem Saugelement gehalten wird, und wobei die Descemet-Membran nach oben von dem schalenförmigen Teil weg weist.

14. Set nach Anspruch 13, bei dem Vorrichtungen vorgesehen sind, um mindestens die Descemet-Membran in ein Fluid einzutauchen.

15. Set nach Anspruch 13 oder 14, bei dem der schalenförmige Teil mindestens teilweise eine lichtreflektierende Oberfläche aufweist.

16. Ausrüstungssatz mit Teilen, die ein Set nach einem der Ansprüche 13-16 und eine Stanzvorrichtung zum Schneiden einer Reißlinie in die Descemet-Membran enthalten, wobei die Reißlinie in einem geringen Abstand von dem äußeren Rand der Descemet-Membran verläuft.

17. Verfahren zum Entfernen einer Descemet-Membran aus einem Auge von außerhalb eines menschlichen oder tierischen Körpers her, mit folgenden Schritten:
- Vornehmen mindestens eines Einschnitts in oder nahe der Linse des Auges in die vordere Kammer;
- Einführen des Endteils einer Vorrichtung nach einem der Ansprüche 1-10 durch die besagte Öffnung in die vordere Kammer und Ziehen der Descemet-Membran zu der Öffnung hin, Lösen der Membran von der Cornea oder Stroma; und
- Entfernen der Descemet-Membran aus der vorderen Kammer durch die Öffnung hindurch.

18. Verfahren nach Anspruch 17, ferner mit dem Schritt des Einführens in eines Fluids, insbesondere eines Gases, vorzugsweise Luft, in die vordere Kammer vor oder während des Lösens der Descemet-Membran.

## Revendications

1. Dispositif (1) destiné à être utilisé en chirurgie oculaire, comprenant une poignée (2) avec, au niveau d'une extrémité, un élément de raclage (3), ledit élément de raclage (3) ayant une pointe (5) et une partie intermédiaire (4) entre ladite partie de raccordement (7) et ladite pointe (5), ladite partie de raccordement (7) comprenant un angle (β) avec la partie intermédiaire, **caractérisé par** ladite pointe (5) qui comprend un angle (α) avec une direction générale de ladite partie intermédiaire (4) compris entre 10 et 80 degrés.

2. Dispositif selon la revendication 1, dans lequel ledit angle (α) est compris entre 25 et 65 degrés, plus spécifiquement entre 35 et 55 degrés et de préférence approximativement 45 degrés.

3. Dispositif selon la revendication 1 ou 2, dans lequel la partie intermédiaire (4) est sensiblement droite et en vue de dessus, colinéaire avec la poignée (2), la pointe (5) s'étendant dans la direction axiale de ladite poignée (2) et de la partie intermédiaire (4).

4. Dispositif selon l'une quelconque des revendications précédentes, dans lequel la longueur de la pointe (5) entre la partie intermédiaire (4) et l'extrémité libre (9) opposée de la pointe (5) est sensiblement plus petite que la longueur de la partie intermédiaire (4) entre la pointe (5) et la partie de raccordement (7), de préférence non supérieure à un tiers de ladite longueur, encore de préférence inférieure d'un cinquième à un dixième.

5. Dispositif selon l'une quelconque des revendications précédentes, dans lequel la pointe (5) a un bord semi-taillant (9), de préférence arrondi.

6. Dispositif selon l'une quelconque des revendications précédentes, dans lequel ladite pointe (5) est courbée dans une première direction, par rapport à la partie intermédiaire (4), le côté opposé d'au moins la partie intermédiaire (4) étant lisse, ayant de préférence des bords longitudinaux arrondis.

7. Dispositif selon l'une quelconque des revendications précédentes, dans lequel la pointe (5) et la partie intermédiaire (4) ont une largeur inférieure à 5 mm, plus spécifiquement inférieure à 4 mm et de préférence inférieure à 3 mm.

8. Dispositif selon l'une quelconque des revendications précédentes, pour retirer une membrane de Descemet d'un oeil par une ouverture, dans une sphère externe dudit oeil, dans lequel ledit élément de raclage (3) en vue de dessus a une longueur entre la courbure (6) située à la partie de raccordement (7) et l'extrémité (9) de la pointe (5) qui est inférieure à la longueur de la chambre antérieure (15) d'un oeil humain.

9. Dispositif (1) selon l'une quelconque des revendications précédentes, dans lequel on prévoit des moyens (10 - 13) pour introduire un fluide, en particulier de l'air à proximité de ladite pointe (5), comprenant un tube (10) avec une ouverture (11) à proximité de ladite pointe (5), ledit tube (10) pouvant être raccordé à une alimentation de fluide (13).

10. Dispositif (1) selon la revendication 9, dans lequel un élément de commande (12) est prévu pour, pendant l'utilisation, forcer ledit fluide à travers ledit tube (10).

11. Ensemble d'un dispositif (1) selon l'une quelconque des revendications 1 à 8, et d'un dispositif pour introduire un fluide tel que de l'air dans une chambre intérieure (15) d'un oeil .

12. Ensemble d'un dispositif (1) selon l'une quelconque des revendications 1 à 10 ou d'un ensemble selon la revendication il et d'un dispositif de visualisation (39), ledit dispositif de visualisation comprenant une extrémité élastique souple pour l'introduction dans une chambre intérieure d'un oeil par un tunnel scléro-cornéen, ladite extrémité étant prévue avec au moins une surface de réflexion de lumière pour réfléchir la lumière à travers la cornée dudit oeil.

13. Ensemble d'un dispositif (1) selon l'une quelconque des revendications 1 à 10 ou d'un ensemble selon la revendication 11, et d'un dispositif de support pour supporter une partie d'un oeil, comprenant une partie d'une cornée et la membrane de Descemet, ledit dispositif de support étant prévu avec une partie en forme de bol et un élément d'aspiration dans ledit élément en forme de bol, de sorte que ladite partie dudit oeil peut être positionnée dans ledit élément en forme de bol, maintenue par ledit élément d'aspiration, la membrane de Descemet étant orientée vers le haut, à distance dudit élément en forme de bol.

14. Ensemble selon la revendication 13, dans lequel on prévoit des moyens pour immerger ladite membrane de Descemet dans un fluide.

15. Ensemble selon la revendication 13 ou 14, dans lequel ladite partie en forme de bol est au moins partiellement prévue avec une surface de réflexion de lumière.

16. Ensemble de pièces composant un ensemble selon l'une quelconque des revendications 13 à 15 et d'un dispositif de poinçonnage pour découper une ligne de coupe dans ladite membrane de Descemet, ladite ligne de coupe s'étendant à une petite distance du bord externe de ladite membrane de Descemet.

17. Procédé pour retirer une membrane de Descemet d'un oeil à l'extérieur du corps humain ou animal, comprenant les étapes consistant à :
pratiquer au moins une incision dans ou à proximité du cristallin dudit oeil, dans la chambre antérieure ;
insérer la pointe d'un dispositif selon l'une quelconque des revendications 1 à 10 par ladite ouverture dans ladite chambre antérieure et tirer la membrane de Descemet vers ladite ouverture, détacher ladite membrane de la cornée ou du stroma ; et
retirer ladite membrane de Descemet de ladite chambre antérieure par ladite ouverture.

18. Procédé selon la revendication 17, comprenant en outre l'étape consistant à introduire un fluide, en particulier un gaz, de préférence de l'air dans ladite chambre antérieure avant ou pendant le détachement de ladite membrane de Descemet.
